Europäisches Patentamt

(19) European Patent Office

Office européen des brevets

(11) Numéro de publication : **0 107 231**
**B1**

(12) **FASCICULE DE BREVET EUROPÉEN**

(45) Date de publication du fascicule du brevet :
**27.05.87**

(51) Int. Cl.⁴ : **A 61 B 17/12**

(21) Numéro de dépôt : **83201408.8**

(22) Date de dépôt : **03.10.83**

(54) **Dispositif d'occlusion partielle d'un vaisseau en particulier de la veine cave caudale.**

(30) Priorité : **21.10.82 FR 8217766**

(43) Date de publication de la demande :
**02.05.84 Bulletin 84/18**

(45) Mention de la délivrance du brevet :
**27.05.87 Bulletin 87/22**

(84) Etats contractants désignés :
**AT BE CH DE GB IT LI LU NL SE**

(56) Documents cités :
**US-A- 3 538 917**
**US-A- 3 730 186**

(73) Titulaire : **Claracq, Michel**
**17, rue de Vittel**
**F-31300 Toulouse (FR)**

(72) Inventeur : **Claracq, Michel**
**17, rue de Vittel**
**F-31300 Toulouse (FR)**

(74) Mandataire : **Barre, Philippe**
**Cabinet Barre-Gatti-Laforgue 95 rue des Amidonniers**
**F-31069 Toulouse Cédex (FR)**

Jouve. 18. rue St-Denis. 75001 Paris. France

## Description

L'invention concerne un dispositif et un clip d'occlusion partielle d'un vaisseau, permettant en particulier d'obturer partiellement la veine cave caudale (ou veine cave inférieure) d'un malade en vue de supprimer le risque mortel de certaines embolies pulmonaires ; bien entendu, l'invention n'est pas limitée à l'occlusion de la veine cave et s'étend dans le secteur médical, chirurgical ou éventuellement vétérinaire, à l'occlusion de tout vaisseau.

On sait que l'embolie pulmonaire est par sa fréquence et sa gravité un problème médical majeur : elle menace tout malade atteint de phlébite des membres et ce, souvent en dépit de la mise en œuvre de traitements médicaux préventifs ou curatifs. L'origine quasi exclusive des embolies se situe au niveau du réseau veineux des membres inférieurs et du pelvis, avec extension possible de la thrombose dans la veine cave caudale. On a donc eu l'idée depuis longtemps de réaliser une obstruction de la veine cave caudale pour arrêter la migration des caillots ou embolies vers le cœur puis vers les poumons.

Initialement, l'obstruction a été réalisée par ligature simple de la veine cave caudale sous rénale, mais cette technique présente l'inconvénient de provoquer dans tous les cas une thrombose massive du segment veineux en amont de la ligature (provenant du caractère total de l'occlusion).

Aussi, la ligature totale a-t-elle été abandonnée au profit de la réalisation d'un barrage partiel de la veine cave caudale. Deux types de techniques de cette nature sont actuellement en pratique :

soit la mise en place de ce barrage partiel à l'intérieur de la veine (filtre, ombrelle ou autre), technique qui présente l'avantage de ne pas nécessiter une anesthésie générale, mais le grave défaut d'entraîner un risque de thrombose en amont du barrage, pratiquement aussi important que dans le cas de la ligature totale (notamment du fait de l'introduction d'un corps étranger à l'intérieur du vaisseau),

soit la mise en place autour de la veine d'un organe externe, dit extra-veineux, qui chevauche ladite veine et cloisonne la lumière interne de celle-ci en plusieurs canaux de plus petits calibres.

La présente invention vise une technique de ce dernier type dans laquelle l'occlusion partielle de la veine ou plus généralement du vaisseau est réalisée à partir de l'extérieur de ce dernier, sans introduction d'un corps étranger à l'intérieur de celui-ci. Un avantage essentiel de cette technique réside dans la diminution sensible des risques de thrombose dont elle s'accompagne.

Le dispositif le plus utilisé et le plus perfectionné pour mettre en œuvre cette technique extra-veineuse est à l'heure actuelle constitué par un clip notamment « clip d'Adams et de Weese » mis au point en 1966 ; ce clip moulé en polytétrafluoroéthylène comporte deux branches en U dont l'une est dentelée. Ce clip est mis en place sur la veine de façon à ce que ses branches chevauchent celle-ci, puis est fermé définitivement au moyen d'un fil qui ligature lesdites branches à leurs extrémités libres (extrémités dotées de gorges à cet effet). Les parois de la veine sont ainsi déformées et la présence des dents confère à la lumière interne de la veine une forme cloisonnée, partiellement obturée, apte à arrêter les caillots ou emboles de grosses tailles tout en laissant le sang circuler dans les canaux de plus petits calibres (qui sont délimités par les dents du clip). Est ainsi assurée de façon satisfaisante, la prévention des embolies pulmonaires dangereuses.

Toutefois, un tel dispositif présente un grave inconvénient.

En effet, sa mise en place doit être considérée comme définitive, car une nouvelle intervention chirurgicale pour enlever le clip deviendrait beaucoup plus risquée : outre les risques habituels d'une anesthésie générale et les risques thrombogènes de toute intervention, elle s'opérerait sur des tissus très remaniés et exposerait le patient notamment à des risques de blessures veineuses graves. De plus si, malgré ces risques, le clip était enlevé au terme de la période embolique, il ne serait alors pratiquement plus envisageable de procéder à une troisième intervention pour en replacer un autre dans le cas où un épisode pathologique ultérieur prescrirait à nouveau la mise en place d'une occlusion partielle : le malade (constituant un terrain particulièrement vulnérable) se trouverait alors sans défense contre un accident embolique (ou obligé de recourir à une technique d'occlusion intra-veineuse avec ses inconvénients propres).

Dans ces conditions, le clip posé de façon définitive sur le malade forme un barrage partiel mais définitif que le malade porte toute sa vie. Or, on a pu constater que ce barrage expose le malade à des risques liés à la stase sanguine permanente qui persiste en amont du clip tant que ce dernier est en place (c'est-à-dire bien après la période embolique pendant toute la vie du malade) : syndrome d'insuffisance veineuse chronique des membres inférieurs et menace de thrombose tardive. C'est là le défaut majeur du clip d'Adams et de Weese et des clips analogues existants actuellement.

La présente invention se propose de remédier à ce défaut et de fournir un dispositif perfectionné d'occlusion partielle, faisant appel à une technique extraveineuse.

L'objectif essentiel de l'invention est donc de fournir un dispositif capable de prévenir les embolies pulmonaires pendant la période embolique, tout en permettant de supprimer les risques tardifs précités, après cette période.

A cet effet, l'invention se propose de fournir un dispositif apte à assurer une occlusion partielle temporaire d'un vaisseau et à permettre la suppression de cette occlusion au moment choisi, et ce, sans intervention profonde (sans avoir, en particulier, à faire subir au patient une anesthésie générale et sans avoir à travailler à nouveau au niveau de la veine cave sur des tissus remaniés).

Un autre objectif de l'invention est de permettre après suppression de l'effet d'occlusion, de renouveler cet effet à la demande, autant de fois que cela peut s'avérer nécessaire, en particulier en cas de nouvel épisode pathologique et ce, comme précédemment, sans intervention profonde.

Un autre objectif de l'invention est de faciliter l'opération de mise en place de l'organe d'occlusion sur le vaisseau.

A cet effet, le dispositif d'occlusion partielle visé par l'invention et défini dans la revendication 1 est du type comprenant un clip muni de dents, adapté pour pouvoir chevaucher extérieurement le vaisseau afin d'en cloisonner la lumière interne en plusieurs canaux ; selon la présente invention, les dents du clip sont rétractiles et associées à des moyens de commande à distance, adaptés pour permettre de les mouvoir entre une position active où lesdites dents en saillie sont aptes à assurer le cloisonnement du vaisseau, et une position passive, où elles se trouvent escamotées sans effet sensible sur le vaisseau. La revendication 20 définit par ailleurs un clip d'occlusion partielle en soi.

De façon connue en soi, le clip est en particulier formé de deux branches rigides ou semi-rigides agencées en U avec une partie coudée pour présenter la forme d'un diapason, lesdites branches comportent des parois d'appui, situées en regard et destinées à venir au contact du vaisseau ; selon des caractéristiques d'un mode de réalisation particulier de la présente invention :

au moins l'une des branches du clip est creuse et forme une chambre interne,

la paroi d'appui de chaque branche creuse comporte des lumières,

au moins une membrane souple est associée aux branches de façon à obturer les lumières des parois d'appui, la ou lesdites membranes étant adaptées pour pouvoir se déployer à travers les lumières, par gonflement, en vue de constituer les dents en position active ou pour pouvoir se dégonfler et s'effacer dans la paroi d'appui en vue de fournir la position passive desdites dents,

les moyens de commande à distance comprennent des moyens de mise en pression ou dépression de la chambre interne de chaque branche creuse, adaptés pour engendrer à la commande le gonflement ou le dégonflement des dents.

Ainsi, en cas de risques d'embolies pulmonaires, le chirurgien met en place le clip conforme à l'invention, dans une situation analogue à un clip d'Adams et De Weese ; au cours de la mise en place, les dents du clip sont en position passive (rétractée) ce qui facilite le glissement du clip à cheval sur le vaisseau. Le chirurgien engendre ensuite le déploiement des dents en agissant sur les moyens de commande à distance de sorte que la fonction d'occlusion partielle se trouve remplie, la veine étant cloisonnée par les dents en saillie, en plusieurs canaux parallèles de plus petits calibres.

Au terme de la période embolique, il suffit d'agir à nouveau sur les moyens de commande à distance pour engendrer la rétraction des dents vers leur position passive, sans avoir à ôter le clip et, en conséquence, sans intervention profonde au niveau de la veine cave. L'effet de cloisonnement est alors supprimé et la stase en amont de la veine est entièrement éliminée, de même que tous les risques ultérieurs dont elle s'accompagne.

Si par la suite, le patient n'est soumis à aucun risque nouveau d'embolie, le clip sera laissé dans son état passif qui permet une circulation sanguine normale sans stase amont. Si au contraire un nouvel épisode pathologique conduisait à une nouvelle apparition de risques d'embolie, les moyens de commande à distance seraient à nouveau actionnés pour disposer les dents dans leur position active déployée, et ce, jusqu'à la fin de cette nouvelle période embolique.

La présente invention s'étend à tout type de dents rétractiles et à tout type de moyens de commande à distance. Toutefois, le mode de réalisation à membranes avec déploiement par pression et rétraction par dépression paraît être avantageux en raison de la simplicité structurelle à laquelle il conduit et de la fiabilité de son fonctionnement.

Dans ce mode de réalisation, les moyens de commande à distance comprennent, de préférence, un cathéter connecté à la chambre interne de chaque branche creuse, et un organe hydraulique ou pneumatique connecté à l'autre extrémité dudit cathéter et adapté pour engendrer la mise en pression ou dépression des chambres internes.

En particulier, l'organe hydraulique ou pneumatique ci-dessus évoqué peut être constitué, d'une part, par un réservoir déformable de fluide, de capacité variable, agencé pour former avec le cathéter et les chambres internes du clip un circuit de fluide fermé, d'autre part, par des moyens de maintien dudit réservoir dans au moins deux états, l'un de plus forte capacité correspondant au dégonflement des dents et à leur position passive, l'autre de plus faible capacité correspondant au gonflement desdites dents et à leur position active.

Le fluide utilisé est de préférence un liquide en raison de son caractère incompressible ; le choix d'un liquide radio-opaque permet de repérer aisément le clip en place par un simple examen radiologique et de déterminer à tout moment quel est l'état des dents (position active ou passive) par l'épaisseur du contraste observé.

L'organe hydraulique (ou éventuellement pneumatique) de commande à distance peut être logé sous la peau du patient (à la manière d'un boîtier de stimulateur cardiaque dit « pace-maker ») au moment de la mise en place du clip ; il peut être actionné ultérieurement pour commander la rétraction des dents ou un nouveau déploiement de celles-ci, par une petite intervention superficielle sous anesthésie locale, sans opération au niveau de la veine cave ni manipulation du clip en place.

Par ailleurs, selon une autre caractéristique d'un mode de réalisation particulier de l'invention, le clip comprend une barrette de fermeture, s'articulant à l'extrémité libre d'une branche et pourvue d'un organe d'encliquetage apte à venir coopérer avec un organe conjugué situé à l'extrémité libre de l'autre branche, en vue de fermer le clip.

La fermeture du clip est ainsi beaucoup plus facile et rapide que de celle des clips antérieurs exigeant une ligature.

Selon un autre mode de réalisation, il est également possible de réaliser la fermeture du clip au moyen d'une ou plusieurs dents de fermeture, situées à proximité des extrémités libres des branches et adaptées pour subir un gonflement en même temps que les autres dents ; chaque dent de fermeture est formée par une membrane en un matériau souple de nature à n'opposer qu'une résistance négligeable au gonflement, cependant que les membranes formant les autres dents comprennent une couche élastique interne, opposant une résistance au gonflement, et une couche inextensible externe limitant le volume de déploiement desdites dents. Par l'établissement d'une pression appropriée dans les chambres internes du clip, il est ainsi possible d'obtenir le dégonflement des dents fonctionnelles, tout en conservant les dents de fermeture en position gonflée, donc fermée.

L'invention s'étend aux parties constitutives du dispositif décrit et, en particulier, à un nouveau clip d'occlusion, comportant des dents rétractiles et, le cas échéant, une barrette de fermeture encliquetable ou des dents de fermeture.

L'invention exposée ci-dessus dans sa forme générale sera mieux comprise à la lecture de la description qui suit et à l'examen des dessins annexés, qui en présentent à titre d'exemples non limitatifs des modes de réalisation ; sur ces dessins qui font partie intégrante de la présente description :

la figure 1 est une vue en perspective d'un premier mode de réalisation d'un dispositif d'occlusion partielle conforme à l'invention,

la figure 2 est une coupe longitudinale par un plan axial A du clip de ce dispositif,

les figures 3 et 4 sont des coupes transversales par des plans BB et CC de ce clip,

la figure 5 est une coupe longitudinale par un plan axial D de l'organe de commande à distance du dispositif,

les figures 6a, 6b et 6c présentent en perspective schématique les trois états fonctionnels du clip,

la figure 7 présente, en coupe transversale, vu de dessous, le dispositif en place dans un corps humain,

les figures 8a et 8b montrent en coupes schématiques le dispositif en place, respectivement dents rétractées en position passive, et dents déployées en position active avec les états correspondants de la veine cave,

la figure 9 est une vue en coupe d'une variante du clip,

la figure 10 présente en perspective un autre mode de réalisation de clip,

la figure 11 est une vue en coupe axiale de ce mode de réalisation,

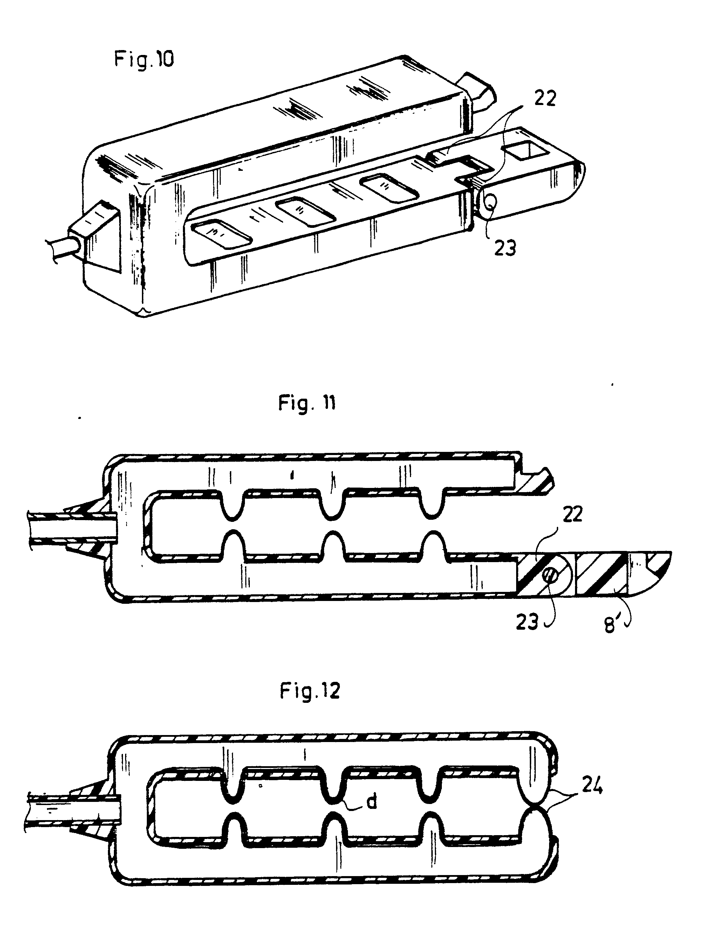

enfin, la figure 12 est une vue en coupe axiale d'un autre mode de réalisation.

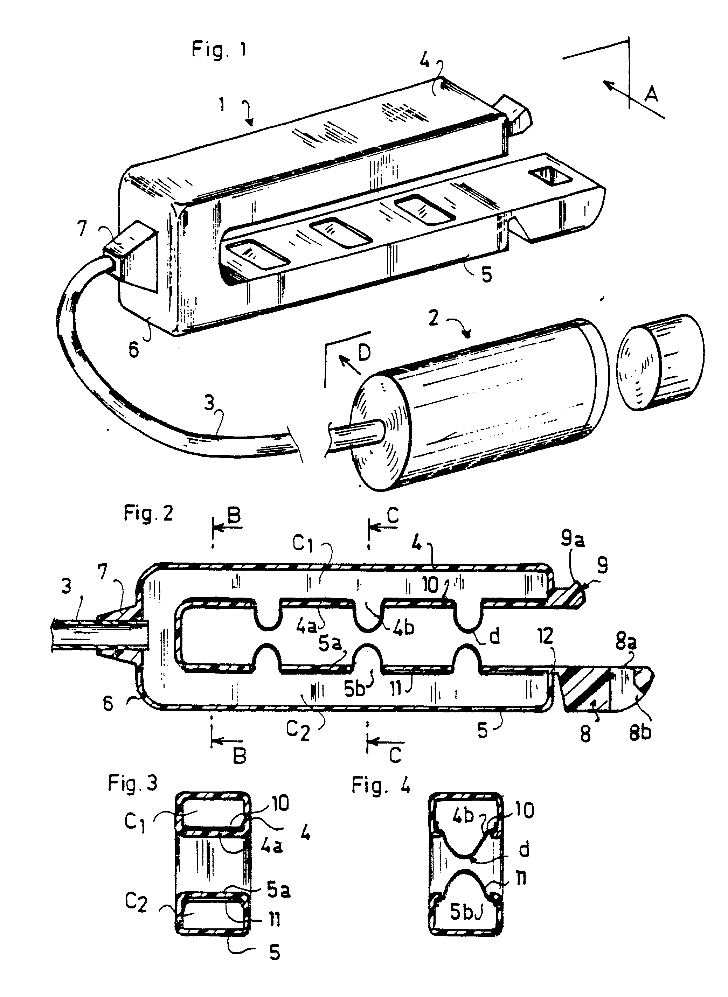

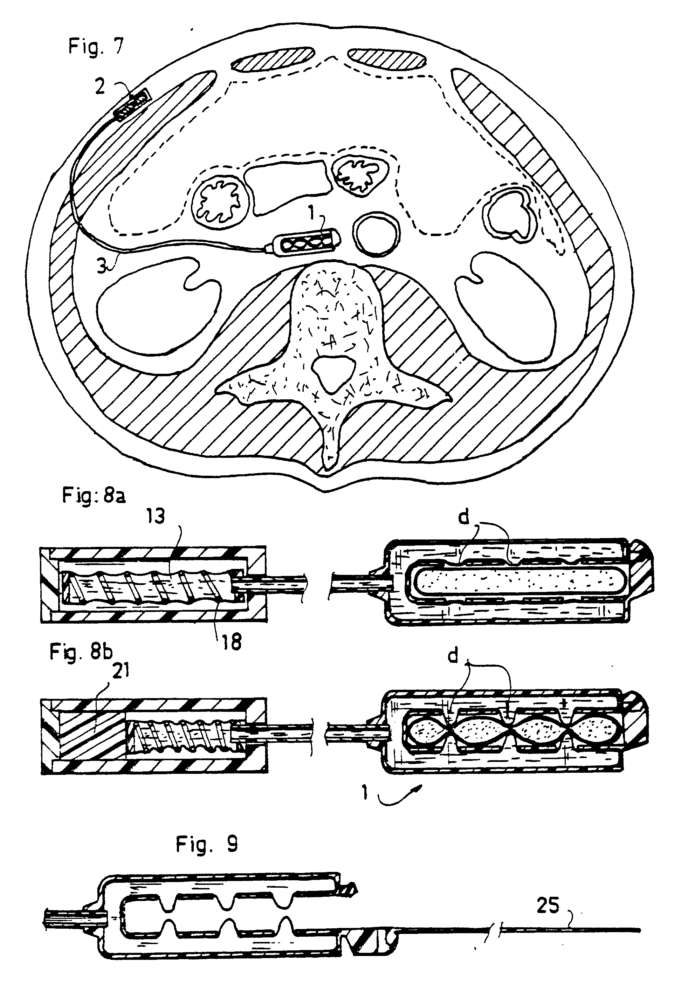

Il est à noter que, à l'exception de la figure 7, les figures sont dessinées à échelle dilatée ; pour illustrer les dimensions, les coupes des figures 2, 3, 4, 5, 11 et 12 ont été dessinées avec une échelle légèrement inférieure à 4.

Le dispositif d'occlusion partielle représenté à titre d'exemple aux figures 1, 2, 3, 4 et 5 comprend essentiellement trois ensembles fonctionnels : un clip 1 appelé à chevaucher un vaisseau en particulier la veine cave caudale (ou inférieure) d'un patient, un organe hydraulique de commande 2 et un cathéter 3 reliant le clip 1 et l'organe hydraulique 2.

Le clip 1 comprend deux branches creuses 4 et 5 qui s'étendent parallèlement et sont réunies par une partie creuse coudée 6, de façon à conférer à l'ensemble la forme d'un diapason en U.

Chaque branche creuse présente une section sensiblement rectangulaire comme le montre la figure 3 et délimite une chambre interne C1 ou C2, ces chambres communiquant par la partie creuse coudée 6 et étant fermées à l'autre extrémité.

La partie coudée 6 comprend en bout, une lumière associée à un embout de connection 7 auquel est relié le cathéter 3 ; la liaison peut être réalisée par collage.

En outre, à l'extrémité libre des branches 4 et 5 sont prévus des moyens de fermeture 8 et 9 du clip qui seront décrits plus loin.

Les branches 4 et 5 comprennent des parois d'appui 4a et 5a qui sont sensiblement planes et sont appelées à venir extérieurement au contact de la paroi de la veine cave, lors de la mise en place du clip, à cheval sur celle-ci.

Ces parois d'appui 4a et 5a sont dotées de lumières telles que 4b et 5b, en l'exemple au nombre de trois par branche, régulièrement réparties le long de celles-ci pour former quatre intervalles à peu près égaux. Ce nombre de lumières peut évidemment être différent, notamment deux ou quatre par branche selon les dimensions du clip (lesquelles sont adaptées à la taille du vaisseau à équiper).

Le clip ci-dessus décrit peut être moulé en matière synthétique biocompatible (polytétrafluoroéthylène ou autre) avec des épaisseurs de matière, telles que chaque branche présente une bonne rigidité, tout en gardant la faculté de légèrement s'écarter ou se rapprocher de l'autre branche, du fait de l'élasticité conférée par la forme du clip.

Par ailleurs, chaque branche comprend, collée intérieurement sur sa paroi d'appui, une membrane souple 10 ou 11, qui obture les lumières précitées de façon à rendre les chambres C1 et C2 étanches. Chaque membrane 10 ou 11 présente au niveau de chaque lumière une forme lui permettant soit d'être gonflée pour former une dent en saillie telle que -d- (figures 2 et 4) soit d'être dégonflée pour venir s'escamoter au niveau de la paroi d'appui 4a ou 5a correspondante (figure 1).

En l'exemple décrit, chaque membrane est réalisée en un matériau souple, non extensible de façon que le volume d'expansion des dents soit limité.

De plus, les lumières 4b et 5b des deux branches sont situées en regard de façon à former des dents -d- disposées deux à deux en regard ; au niveau des dents, la couche souple inextensible de chaque membrane est adaptée de sorte que les sommets des paires de dents en regard viennent, en position active déployée, se situer au voisinage l'un de l'autre, sans contact l'un avec l'autre. La distance entre les sommets des dents déployées est prévue à peu près égale au double de l'épaisseur de la paroi du vaisseau à équiper de façon à éviter des nécroses locales par écrasement desdites parois.

Les dimensions du clip conforme à l'invention peuvent être du même ordre que celles d'un clip d'Adams et De Weese ; toutefois, la largeur des branches est de préférence prévue égale à environ 5 mm de façon que le clip une fois mis en place, présente une bonne stabilité (sans risque de rotation sur lui-même), tout en étant facile à loger dans l'espace rétropéritonéal, à l'avant de la colonne vertébrale.

Par ailleurs, les moyens de fermeture du clip comprennent, d'une part, une barrette de fermeture 8 qui s'articule à l'extrémité libre de la branche 5 et comporte un organe d'encliquetage 8a, d'autre part, un organe conjugué 9 situé à l'extrémité libre de l'autre branche.

En l'exemple, l'organe 9 est constitué par un téton pourvu d'un becquet supérieur 9a et la barrette 8 comprend une lumière 8a agencée pour recevoir le téton 9 lorsque ladite barrette est rabattue vers l'autre branche. Cette lumière 8a présente une dimension permettant, sous l'effet d'une pression d'encliquetage, le passage du téton 9 et de son becquet 9a et possède un évasement 8b où ce becquet 9a vient se loger en fin d'encliquetage.

De tels moyens permettent au chirurgien de fermer de façon rapide et facile, le clip en fin de mise en place sur la veine, afin d'immobiliser celui-ci, sans risque de déplacement accidentel.

Il est à noter que ces moyens de fermeture sont adaptés pour bloquer parfaitement les deux branches l'une par rapport à l'autre de façon qu'elles ne puissent rigoureusement pas s'écarter ou se rapprocher.

Dans le mode de réalisation représenté aux figures 1 et 2, la barrette de fermeture 8 est articulée sur la branche 5 par un pontet souple 12 qui prolonge ladite barrette 8 et se rattache à ladite branche 5. L'épaisseur de ce pontet est prévue pour lui donner l'élasticité nécessaire, compte tenu du matériau utilisé.

Cette forme de réalisation permet de fabriquer d'un seul tenant, par moulage, les branches et les moyens de fermeture.

Par ailleurs, le deuxième ensemble fonctionnel du dispositif conforme à l'invention est constitué par le cathéter 3, classique en lui-même, et de longueur au moins égale à 30 cm, adaptée à la taille du malade.

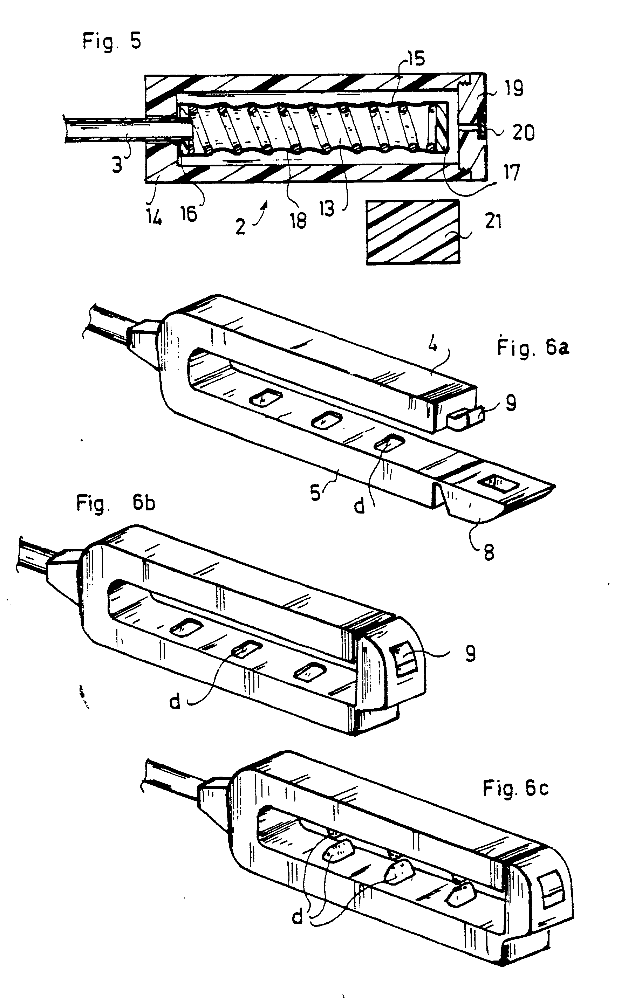

Un mode de réalisation de l'organe hydraulique 2 qui constitue le troisième ensemble fonctionnel du dispositif est représenté à la figure 5.

Cet organe hydraulique comprend un réservoir déformable 13 qui est logé dans un boîtier 14. Ce réservoir 13 est formé par une membrane 15 de forme générale cylindrique, fermée à ses deux bouts par deux petits disques rigides 16 et 17 et apte à se déployer ou se replier longitudinalement. Cette membrane contient un ressort 18 qui la guide et la sollicite dans le sens de son déploiement.

Le disque 16 est percé d'une lumière à laquelle est étanchement connectée, notamment par collage, l'extrémité du cathéter 3 (qui traverse le boîtier 14 par un perçage approprié).

A son autre extrémité, le boîtier 14 est équipé d'un couvercle 19 qui est vissé sur celui-ci et forme un fond démontable ; ce couvercle est muni d'un clapet 20 permettant l'évacuation de l'air intérieur au boîtier, tout en évitant l'entrée du sang ou autre liquide dans celui-ci.

Le ressort 18 a tendance à maintenir le réservoir dans son état de plus forte capacité (correspondant à la position passive des dents du clip). Des moyens de blocage sont par ailleurs associés audit réservoir pour pouvoir le maintenir dans un état de faible capacité, avec compression du ressort 18. En l'exemple, ces moyens sont très simplement constitués par une câle cylindrique amovible 21 que le chirurgien peut insérer dans le boîtier entre le fond 19 de celui-ci et le disque 17 du réservoir.

Le réservoir 13, le cathéter 3 et les chambres C1 et C2 des branches 4, 5 forment un circuit fermé qui est rempli de liquide radio-opaque. Le réservoir est dimensionné de façon que, en l'absence de la câle 21, les dents -d- du clip se trouvent escamotées en position passive (état flasque) au niveau des parois d'appui. Lorsque la câle 21 est mise en place, les dents sont mises en pression et viennent se disposer en position active.

Il est à noter que plusieurs tailles de clips 1 peuvent être associées à un seul type d'organe hydraulique 2 tel que ci-dessus décrit ; il suffit d'équiper cet organe d'un jeu de câles de longueurs adaptées pour assurer une mise en pression appropriée des dents en fonction du nombre de celles-ci (et de la capacité des chambres C1 et C2 du clip).

Les figures 6a, 6b et 6c présentent le clip conforme à l'invention, respectivement :

dans la position de mise en place sur la veine, dents -d- escamotées et barrette 8 dans le prolongement de la branche 5,

après mise en place mais avant manœuvre de l'organe hydraulique, dents -d- escamotées et barrette 8 rabattue et encliquetée sur le téton 9, après manœuvre de l'organe hydraulique, dents -d- en saillie.

La figure 7 schématise le clip 1 en place sur la veine cave inférieure d'un malade en amont de l'abouchement des veines rénales ; l'organe hydraulique 2 est logé sous les téguments de façon à pouvoir être ultérieurement facilement manœuvré par une intervention légère sous anesthésie locale.

Après la mise en place du clip, le chirurgien manœuvre l'organe hydraulique 2 en insérant la câle 21 dans son boîtier : le clip se trouve alors en position d'occlusion partielle comme le schématise la figure 8b ; la lumière interne de la veine cave est divisée en plusieurs canaux de plus petits calibres et les risques d'embolies dangereuses sont écartés pour le malade.

Au terme de la période embolique, l'organe hydraulique 2 est manœuvré en sens inverse (en ôtant la câle 21) et les dents -d- sont ramenées au niveau des parois d'appui par la dépression qui apparaît dans les chambres C1 et C2 des branches (figure 8a).

Il est à noter que le retour du réservoir 13 vers son état de forte capacité s'opère progressivement sous l'action du ressort 18 de sorte que le retour des dents en position passive s'opère lui-même progressivement ; le module élastique de ce ressort 18 est prévu pour garantir ce retour (c'est-à-dire pour être apte à vaincre les forces d'adhérence des parois de la veine cave) tout en assurant ledit retour de façon assez lente pour éviter des traumatismes éventuels de la veine.

On conçoit l'intérêt de l'invention qui permet au terme de la période embolique de supprimer les effets du clip 1 sans avoir à enlever ou manipuler celui-ci.

Bien entendu, les ensembles constituant le dispositif de l'invention peuvent revêtir d'autres formes de réalisation.

Par exemple, la figure 9 représente un clip similaire au précédent mais comportant une languette élastique de mise en place 25, qui se rattache à l'extrémité libre de sa barrette de fermeture. Cette languette dont la longueur est d'environ 15 cm (ou davantage) est destinée à faciliter la mise en place du clip ; elle est sectionnée une fois le clip en place et refermé.

Par ailleurs, les figures 10 et 11 représentent un clip similaire aux précédents mais dans lequel la barrette de fermeture 8' est une pièce rapportée, articulée sur la branche correspondante par une chape 22 et un axe d'articulation 23.

En outre, la figure 12 représente un autre mode de réalisation, dans lequel la fermeture du clip est engendrée par deux dents spéciales 24, dites dents de fermeture, qui sont situées en regard l'une de l'autre, à proximité de l'extrémité libre des branches du clip.

Ces dents de fermeture 24 sont formées d'une façon analogue aux dents fonctionnelles -d- par des membranes souples qui peuvent se gonfler ou se dégonfler au niveau de lumières des parois d'appui.

En l'exemple, les membranes formant les dents de fermeture sont constituées par une couche d'un matériau non extensible et non élastique de façon à n'opposer qu'une résistance négligeable au gonflement ; à l'état gonflé, ces dents de fermeture viennent en appui l'une contre l'autre et remplissent la fonction de fermeture que remplissait la barrette 8 dans les modes de réalisation précédents.

Les membranes formant les autres dents (dents fonctionnelles -d-) sont constituées par deux couches superposées : une couche élastique interne apte à opposer une résistance au gonflement, et une couche inextensible externe apte à limiter, comme précédemment, le volume de déploiement desdites dents.

L'organe hydraulique est analogue à celui déjà décrit mais, pour chaque taille de clip, lui sont associées deux câles de longueurs différentes de façon à définir trois états de pression du dispositif :

un état de dépression du clip, où toutes les dents sont escamotées (équivalent à l'état de la figure 6a),

un état de pression partielle du clip, où seules les dents de fermeture 24 sont en saillie, la pression étant insuffisante pour vaincre la résistance élastique des autres dents (état équivalent à l'état de la figure 6b).

enfin, un état de pression complète du clip, où la pression est suffisante pour déployer les dents fonctionnelles -d- (état équivalent à l'état de la figure 6c).

Au terme d'une période embolique, la câle la plus longue est remplacée par la câle la plus courte de sorte que le clip passe du troisième état au deuxième. Le premier état ne sert que pour la mise en place du clip (ou très rarement pour son retrait dans le cas d'une exceptionnelle prescription d'ablation de celui-ci).

Par ailleurs il est à noter que le dispositif de l'invention peut être utilisé comme régulateur de flux vasculaire à action progressive. Il suffit de prévoir des moyens de commande à distance, adaptés pour permettre de déplacer de façon progressive les dents entre leur position dite active, où elles sont entièrement en saillie, et leur position passive. Ce déplacement peut être réalisé de façon continue ou de façon discrète, par pas (en particulier au moyen d'un jeu de cales dans le mode de réalisation décrit). Bien entendu, les moyens de commande peuvent être adaptés pour être déclenchés à l'extérieur du corps sans aucune intervention chirurgicale.

**Revendications**

1. Dispositif d'occlusion partielle d'un vaisseau, du type comprenant un clip (1) muni de dents (d), adapté pour pouvoir chevaucher extérieurement le vaisseau afin d'en cloisonner la lumière interne en plusieurs canaux, ledit dispositif d'occlusion partielle étant caractérisé en ce que les dents (d) du clip sont rétractiles et associées à des moyens de commande à distance (2, 3) adaptés pour permettre de les mouvoir entre une position active où lesdites dents (d) en saillie sont aptes à assurer le cloisonnement du vaisseau, et une position passive, où elles se trouvent escamotées sans effet sensible sur le vaisseau.

2. Dispositif d'occlusion partielle selon la revendication 1, comprenant un clip (1) formé de deux branches rigides ou semi-rigides (4, 5) agencées en U avec une partie coudée (6) pour présenter la forme d'un diapason, lesdites branches comportant des parois d'appui (4a, 5a) situées en regard et destinées à venir au contact du vaisseau, ledit dispositif étant caractérisé en ce que :

au moins l'une des branches (4, 5) du clip est creuse et forme une chambre interne (C1, C2),

la paroi d'appui (4a, 5a) de chaque branche creuse comporte des lumières (4b, 5b),

au moins une membrane souple (10, 11) est associée aux branches de façon à obturer les lumières (4b, 5b) des parois d'appui, la ou lesdites membranes étant adaptées pour pouvoir se déployer à travers les lumières, par gonflement, en vue de constituer les dents (d) en position active ou pour pouvoir se dégonfler et s'effacer dans la paroi d'appui en vue de fournir la position passive desdites dents,

les moyens de commande à distance comprennent des moyens (2) de mise en pression ou dépression de la chambre interne de chaque branche creuse, adaptés pour engendrer à la commande le gonflement ou le dégonflement des dents.

3. Dispositif d'occlusion partielle selon la revendication 2, caractérisé en ce que les deux branches (4, 5) du clip sont creuses et forment deux chambres internes (C1, C2) communiquant par la partie coudée (6) du clip, elle-même creuse, chacune des deux parois (4a, 5a) en regard desdites branches comportant des lumières (4b, 5b) telles que précitées et au moins une membrane souple (10, 11) de façon à former des dents sur chacune desdites parois.

4. Dispositif d'occlusion partielle selon la revendication 3, caractérisé en ce que les membranes (10, 11) et les lumières (4b, 5b) des parois d'appui sont agencées de façon à former des dents (d) situées en regard deux à deux.

5. Dispositif d'occlusion selon la revendication 4, caractérisé en ce que les membranes (10, 11) comprennent une couche d'un matériau souple inextensible, adaptée pour que les sommets des paires de dents en regard viennent en position active, se situer au voisinage l'un de l'autre, sans contact l'un avec l'autre.

6. Dispositif d'occlusion partielle selon l'une des revendications 2, 3, 4 ou 5, caractérisé en ce que les moyens de commande à distance comprennent un cathéter (3) connecté à la chambre interne (C1, C2) de chaque branche creuse, et un organe hydraulique ou pneumatique (2) connecté à l'autre extrémité dudit cathéter et adapté pour engendrer la mise en pression ou dépression des chambres internes.

7. Dispositif d'occlusion partielle selon les revendications 3 et 6 prises ensemble, caractérisé en ce que le cathéter (3) est connecté à la partie coudée creuse (6) du clip.

8. Dispositif d'occlusion partielle selon l'une des revendications 6 ou 7, caractérisé en ce que l'organe hydraulique ou pneumatique comprend, d'une part, un réservoir déformable de fluide (13) de capacité variable, agencé pour former avec le cathéter (3) et les chambres internes (C1, C2) du clip un circuit de fluide fermé, d'autre part, des moyens (14, 18, 19, 21) de maintien dudit réservoir dans au moins deux états, l'un de plus forte capacité correspondant au dégonflement des dents (d) et à leur position passive, l'autre de plus faible capacité correspondant au gonflement desdites dents et à leur position active.

9. Dispositif d'occlusion partielle selon la revendication 8, caractérisé en ce que le réservoir (13) est formé par une membrane (15) de forme générale cylindrique, fermée à ses deux bouts et apte à se déployer ou se replier longitudinalement, un ressort (18) étant associé à ladite membrane pour la guider et la solliciter dans le sens de son déploiement en vue de maintenir le réservoir dans son état de plus forte capacité, des moyens de blocage (14, 19, 21) étant associés audit réservoir pour pouvoir le maintenir dans son état de faible capacité, avec compression du ressort (18).

10. Dispositif d'occlusion partielle selon la revendication 9, caractérisé en ce que le réservoir (13) est logé dans un boîtier (14) pourvu d'un fond démontable (19), au moins une câle amovible (21) étant prévue pour pouvoir s'insérer entre le fond du boîtier et le réservoir afin de maintenir celui-ci dans son état de faible capacité.

11. Dispositif d'occlusion partielle selon l'une des revendications 8, 9 ou 10, caractérisé en ce que le circuit fermé de fluide formé par le réservoir (13), le cathéter (3) et les chambres internes (C1, C2) du clip (1) est rempli d'un liquide radio-opaque.

12. Dispositif d'occlusion partielle selon l'une des revendications 1, 2, 3, 4, 5, 6, 7, 8, 9, 10 ou 11, comprenant un clip (1) formé de deux branches rigides ou semi-rigides (4, 5) agencées en U avec une partie coudée (6) pour présenter la forme d'un diapason, ledit dispositif étant caractérisé en ce qu'il comprend une barrette de fermeture (8, 8'), s'articulant à l'extrémité libre d'une branche (5) et pourvue d'un organe d'encliquetage (8a) apte à venir coopérer avec un organe conjugué (9) situé à l'extrémité libre de l'autre branche (4), en vue de fermer le clip.

13. Dispositif d'occlusion partielle selon la revendication 12, caractérisé en ce que les organes d'encliquetage de la barrette (8, 8') et de la branche sont adaptés pour bloquer les deux branches l'une par rapport à l'autre dans le sens de leur écartement et de leur rapprochement.

14. Dispositif d'occlusion partielle selon l'une des revendications 12 ou 13, caractérisé en ce que la barrette (8) est articulée sur la branche (5) par un pontet souple (12) qui la prolonge et se rattache à ladite branche.

15. Dispositif d'occlusion partielle selon l'une des revendications 12 ou 13, caractérisé en ce que la barrette (8') est une pièce rapportée et est articulée sur la branche (5) par une chape (22) et un axe d'articulation (23).

16. Dispositif d'occlusion partielle selon l'une des revendications 12, 13, 14 ou 15, caractérisé en ce que la barrette est dotée, à son extrémité libre, d'une languette souple de mise en place (25).

17. Dispositif d'occlusion partielle selon l'une des revendications 2, 3, 4, 5, 6, 7, 8, 9, 10 ou 11, comprenant un clip (1) formé de deux branches rigides ou semi-rigides (4, 5) agencées en U avec une partie coudée (6) pour présenter la forme d'un diapason, ledit dispositif étant caractérisé en ce que au moins l'une des branches du clip comprend à proximité de son extrémité libre une dent (24) adaptée pour subir un gonflement en vue d'assurer la fermeture du clip.

18. Dispositif d'occlusion partielle selon la revendication 17, caractérisé en ce que les branches (4, 5) du clip comprennent deux dents de fermeture (24) situées en regard et adaptées pour venir en appui l'une contre l'autre lors du gonflement.

19. Dispositif d'occlusion partielle selon l'une des revendications 17 ou 18, caractérisé en ce que chaque dent de fermeture (24) est formée par une membrane en un matériau souple de nature à n'opposer qu'une résistance négligeable au gonflement, cependant que les membranes formant les autres dents (d) comprennent une couche élastique interne apte à opposer une résistance au gonflement, et une couche inextensible externe apte à limiter le volume de déploiement desdites dents.

20. Clip d'occlusion partielle d'un vaisseau, caractérisé en ce qu'il comprend des dents (d) rétractiles adaptées pour pouvoir se mouvoir entre une position active de cloisonnement de la lumière interne du vaisseau en plusieurs canaux et une position passive sans effet sensible sur le vaisseau lorsque ledit clip chevauche extérieurement ledit vaisseau.

21. Clip selon la revendication 20, formé de deux branches rigides ou semi-rigides (4, 5) agencées en U avec une partie coudée (6) pour présenter la forme d'un diapason, ledit clip étant caractérisé en ce qu'il comprend une barrette de fermeture (8, 8'), s'articulant à l'extrémité libre d'une branche (5) et pourvue d'un organe d'encliquetage (8a) apte à venir coopérer avec un organe conjugué (9) situé à l'extrémité libre de l'autre branche (4), en vue de fermer le clip.

22. Clip selon la revendication 20, formé de deux branches rigides ou semi-rigides (4, 5) agencées en U avec une partie coudée (6) pour présenter la forme d'un diapason, ledit clip étant caractérisé en ce que au moins l'une des branches du clip comprend à proximité de son extrémité libre une dent (24) adaptée pour subir un gonflement en vue d'assurer la fermeture du clip.

**Claims**

1. A partial occlusive device for blood vessels of the type comprising a clip (1) provided with teeth (d) capable of exterior application to the blood vessel with a view to subdividing the inner cavity into several channels, the said partial occlusive device being characterised in that the teeth (d) of the clip are retractable and associated with means of remote control (2, 3) capable of being moved between an active position in which the said teeth (d) as they project are able to ensure subdivision of the blood vessel and a passive position in which they are withdrawn without any noticeable effect on the blood vessel.

2. A partial occlusive device in accordance with claim 1, comprising a clip (1) consisting of two rigid or semi-rigid branches (4, 5) arranged in a U with a curved part (6), thus presenting the form of a tuning fork, the said branches comprising support walls (4a, 5a) opposite one another and intended to contact the blood vessel, the said device being characterised in that

at least one of the branches (4, 5) of the clip is hollow and constitutes an internal chamber (C1, C2)

the support wall (4a, 5a) of each hollow branch is provided with apertures (4b, 5b)

at least one flexible membrane (10, 11) is associated with the branches so as to block the apertures (4b, 5b) in the support walls, the said membrane or membranes being capable of extending, as a result of expansion, through the apertures so as to constitute the teeth (d) in the active position or of contracting and withdrawing into the support wall thus giving rise to the passive position of the said teeth.

the means of remote control comprise means (2) for causing the internal chamber of each hollow branch to be pressurised or depressurised, capable of causing on command expansion or contraction of the teeth.

3. A partial occlusive device in accordance with claim 2, characterised in that the two branches (4, 5) of the clip are hollow and form two internal chambers (C1, C2) communicating through the likewise hollow curved part (6) of the clip, each of the two opposite walls (4a, 5a) of the said branches having cavities (4b, 5b) as described above and at least one flexible membrane (10, 11) with a view to forming teeth on each of the said walls.

4. A partial occlusive device in accordance with claim 3, characterised in that the membranes (10, 11) and the apertures (4b, 5b) of the support walls are arranged in such a way as to form teeth (d) located opposite one another two by two.

5. A partial occlusive device in accordance with claim 4, characterised in that the membranes (10, 11) comprise a layer of inextensible flexible material enabling the tips of the pairs of opposite teeth to approach one another in the active position without coming into contact with one another.

6. A partial occlusive device in accordance with any of claims 2, 3, 4 or 5, characterised in that the means of remote control comprise a catheter (3) connected to the internal chamber (C1, C2) of each hollow branch and a hydraulic or pneumatic device (2) connected to the other end of the said catheter and capable of causing the internal chambers to be pressurised or depressurised.

7. A partial occlusive device in accordance with claims 3 and 6 taken jointly, characterised in the catheter (3) is connected to the hollow curved part (6) of the clip.

8. A partial occlusive device in accordance with any of claims 6 or 7, characterised in that the hydraulic or pneumatic device comprises, on the one hand, a deformable fluid reservoir (13) of variable capacity arranged to form together with catheter (3) and the internal chambers (C1, C2) of the clip a closed fluid circuit and, on the other hand, means (14, 18, 19, 21) for maintaining the said reservoir in at least two states, one of higher capacity corresponding to the contraction of teeth (d) and their passive position and the other of lower capacity corresponding to the expansion of the said teeth and their active position.

9. A partial occlusive device in accordance with claim 8 characterised in that reservoir (13) is formed by a membrane (15) of generally cylindrical form closed at its two ends and capable of extending or contracting in the longitudinal direction, a spring (18) associated with the said membrane so as to guide and coax it in the direction of its extension with a view to maintaining the reservoir in its state of higher capacity and blocking means (14, 19, 21) associated with the said reservoir with a view to enabling its maintenance in the state of low capacity, spring (18) being compressed.

10. A partial occlusive device in accordance with claim 9, characterised in that reservoir (13) is located within a housing (14) provided with a base (19) which can be dismantled, at least one removable packing-piece (21) being provided for insertion between the base of the housing and the reservoir with a view to maintaining the letter in its state of low capacity.

11. A partial occlusive device in accordance with any of claims 8, 9 or 10, characterised in that the closed fluid circuit formed by reservoir (13), catheter (3) and the internal chambers (C1, C2) of clip (1) is filled with a radio-opaque liquid.

12. A partial occlusive device in accordance with any of claims 1, 2, 3, 4, 5, 6, 7, 8, 9, 10 or 11, comprising a clip (1) consisting of two rigid or semi-rigid branches (4, 5) arranged in a U with a curved part (6) thus presenting the form of a tuning fork, the said device being characterised in that it comprises a closure element (8, 8') movably joined to the free end of a branch (5) and provided with an engaging device (8a) capable of co-operating with a corresponding device (9) located at the free end of the other branch (4) with a view to closing the clip.

13. A partial occlusive device in accordance with claim 12, characterised in that the engaging devices of the element (8, 8') and the branch are capable of blocking the two branches in relation to one another so that they can neither move apart nor approach one another.

14. A partial occlusive device in accordance with any of claims 12 or 13, characterised in that the element (8) is movably joined to branch (5) by a flexible bridging piece (12) extending the said element and attached to the said branch.

15. A partial occlusive device in accordance with any of claims 12 or 13, characterised in that the element (8') is an added device and movably joined to branch (5) by a shell (22) and an articulation spindle (23).

16. A partial occlusive device in accordance with any of claims 12, 13, 14 or 15, characterised in that the element is provided at its free end with a flexible locating tongue (25).

17. A partial occlusive device in accordance with any of claims 2, 3, 4, 5, 6, 7, 8, 9, 10 or 11 comprising a clip (1) consisting of two rigid or semi-rigid branches (4, 5) arranged in a U with a curved part (6) thus presenting the form of a tuning fork the said device being characterised in that at least one of the branches of the clip has in the vicinity of its free end a tooth (24) capable of undergoing expansion with a view to ensuring closure of the clip.

18. A partial occlusive device in accordance with claim 17 characterised in that branches (4, 5) of the clip comprise two closure teeth (24) located opposite one another and capable of abutting one another during expansion.

19. A partial occlusive device in accordance with any of claims 17 or 18 characterised in that each closure tooth (24) is formed by a membrane of flexible material opposing only a negligible resistance to expansion whereas the membranes forming the other teeth (d) comprise an elastic internal layer capable of resisting expansion and an inextensible external layer capable of limiting the volume by which the said teeth extend.

20. A partial occlusive device for blood vessels characterised in that it comprises retractable teeth (d) capable of being moved between an active position in which they suddivide the internal cavity of the blood vessel into several channels and a passive position without noticeable influence on the blood vessels when the said clip is applied externally to the said vessel.

21. A clip in accordance with claim 20 consisting of two rigid or semi-rigid branches (4, 5) arranged in a U with a curved part (6) thus presenting the form of a tuning fork, the said clip being characterised in that it comprises a closure element (8, 8') movably joined to the free end of one branch (5) and provided with an engaging device (8a) capable of co-operating with a corresponding device (9) located at the free of the other branch (4) with a view to closing the clip.

22. A clip in accordance with claim 20 consisting of two rigid or semi-rigid branches (4, 5) arranged in a U with a curved piece (6) thus presenting the form of a tuning fork, the said clip being characterised in that at least one of the branches of the clip has near its free end a tooth (24) capable of undergoing expansion with a view to ensuring closure of the clip.

**Patentansprüche**

1. Eine teilweise Okkludiereinrichtung für Blutgefäße von eine mit Zähnen (d) vorgesehene Klammer (1) umfassender Art, die so beschaffen ist, daß sie an der Außenseite des Blutgefäßes angebracht werden kann, um dessen Innenraum in mehrere Kanäle zu unterteilen, wobei die besagte teilweise Okkludiereinrichtung für Blutgefäße dadurch gekennzeichnet ist, daß die Zähne (d) der Klammer zurückziehbar und mit Mitteln zur Fernsteuerung (2, 3) verbunden sind, so daß sie zwischen einer aktiven Position, in der die besagten Zähne (d) vorstehen und in der Lage sind die Unterteilung des Blutgefäßes zu gewährleisten, und einer passiven Position, in der sie zurückgezogen sind und keinen merkbaren Einfluß auf das Blutgefäß ausüben, verstellt werden können.

2. Eine teilweise Okkludiereinrichtung im Einklang mit Anspruch 1, mit einer Klammer (1), die aus zwei U-förmig angeordneten steifen oder halbsteifen Schenkeln (4, 5) mit einem gekrümmten Teil (6) besteht, so daß sie die Form einer Stimmgabel hat, wobei die besagten Schenkel einander gegenüberliegende Stützwände (4a, 5a) umfassen, die so beschaffen sind, daß sie mit dem Blutgefäß in Berührung kommen, wobei die besagte Einrichtung dadurch gekennzeichnet ist, daß

mindestens einer der Schenkel (4, 5) der Klammer hohl ist und eine innere Kammer (C1, C2) bildet,

die Stützwand (4a, 5a) jedes Hohlschenkels Hohlräume (4b, 5b) enthält,

die Schenkel mindestens eine flexible Membran (10, 11) umfassen, um die Öffnungen (4b, 5b) der Stützwände zu verschließen, wobei die besagte(n) Membran(en) so beschaffen ist (sind), daß sie sich durch Anschwellung durch die Öffnungen hindurch erstrecken kann (können), um in der aktiven Position Zähne (d) zu bilden, oder daß sie sich entspannen und in die Stützwand zurückziehen kann (können), um die passive Position der besagten Zähne einzunehmen.

Fernsteuerungsmittel Vorrichtungen (2) für die Ausübung oder Entspannung von Druck in der Innenkammer jedes hohlen Schenkels umfassen, die so beschaffen sind, daß sie auf Befehl das Anschwellen oder Entspannen der Zähne bewirken.

3. Eine teilweise Okkludiereinrichtung im Einklang mit Anspruch 2, dadurch gekennzeichnet, daß die beiden Schenkel (4, 5) der Klammer hohl sind und zwei innere Kammern (C1, C2) bilden, die durch den gekrümmten Teil (6) der Klammer, der ebenfalls hohl ist, miteinander in Verbindung stehen, wobei jede der beiden gegenüberliegenden Wände (4a, 5a) der besagten Schenkel Öffnungen der vorstehend beschriebenen Art (4b, 5b) und mindestens eine flexible Membran (10, 11) umfaßt, um an jeder der besagten Wände Zähne zu bilden.

4. Eine teilweise Okkludiereinrichtung gemäß Anspruch 3, dadurch gekennzeichnet, daß die Membranen (10, 11) und die Öffnungen (4b, 5b) der Stützwände so angeordnet sind, daß paarweise einander gegenüber befindliche Zähne (d) entstehen.

5. Eine teilweise Okkludiereinrichtung gemäß Anspruch 4, dadurch gekennzeichnet, daß die Membranen (10, 11) eine Schicht aus einem nicht dehnbaren flexiblen Material umfassen, die so beschaffen ist, daß die Scheitel der paarweise einander gegenüberliegenden Zähne in der aktiven Position einander nahe aber miteinander nicht in Berührung sind.

6. Eine teilweise Okkludiereinrichtung gemäß einem der Ansprüche 2, 3, 4 oder 5, dadurch gekennzeichnet, daß die Fernsteuerungsmittel einen Katheter (3), der an die innere Kammer (C1, C2) jedes hohlen Schenkels angeschlossen ist, sowie eine hydraulische oder pneumatische Vorrichtung (2) umfassen, die an das andere Ende des besagten Katheters angeschlossen und so beschaffen ist, daß in den inneren Kammern Druck ausgeübt bzw. entspannt wird.

7. Eine teilweise Okkludiereinrichtung gemäß der Ansprüche 3 und 6 gemeinsam, dadurch gekennzeichnet, daß der Katheter (3) an den gekrümmten hohlen Teil (6) der Klammer angeschlossen ist.

8. Eine teilweise Okkludiereinrichtung gemäß einem der Ansprüche 6 oder 7, dadurch gekennzeichnet, daß die hydraulische oder pneumatische Vorrichtung einerseits einen verformbaren Flüssigkeitsbehälter (13) veränderlichen Fassungsvermögens umfaßt, der so beschaffen ist, daß er gemeinsam mit dem Katheter (3) und den inneren Kammern (C1, C2) der Klammer einen geschlossenen Flüssigkeitskreis bildet, und andererseits Mittel (14, 18, 19, 21) zur Erhaltung des besagten Behälters in mindestens zwei Zuständen, einem mit höherem Fassungsvermögen, der der Entspannung der Zähne (d) und deren passiver Position entspricht, und einem geringeren Fassungsvermögens, der dem angeschwellten Zustand der besagten Zähne und deren aktiver Position entspricht.

9. Eine teilweise Okkludiereinrichtung im Einklang mit Anspruch 8, dadurch gekennzeichnet, daß der Behälter (13) aus einer Membran (15) allgemein zylindrischer Form besteht, die an ihren beiden Enden geschlossen und in der Lage ist, sich der Länge nach auszudehnen bzw. zusammenzuziehen, sowie einer Feder (18), die mit der besagten Membran zwecks Führung und Betätigung in deren Ausdehnungsrichtung verbunden ist, um den Behälter im Zustand größeren Fassungsvermögens zu erhalten, und Blockiermitteln (14, 19, 21), die mit dem besagten Behälter verbunden sind, um diesen im Zustand geringen Fassungsvermögens zu erhalten, wobei die Feder (18) zusammengedrückt wird.

10. Eine teilweise Okkludiereinrichtung im Einklang mit Anspruch 9, dadurch gekennzeichnet, daß sich der Behälter (13) in einem Gehäuse (14) mit einem abmontierbaren Boden (19) befindet, wobei mindestens ein entfernbares Paßstück (21) vorgesehen ist, das zwischen dem Boden des Gehäuses und dem Behälter eingesetzt werden kann, um diesen im Zustand geringen Fassungsvermögens zu erhalten.

11. Eine teilweise Okkludiereinrichtung gemäß einem der Ansprüche 8, 9 oder 10, dadurch gekennzeichnet, daß der durch den Behälter (13), den Katheter (3) und die inneren Kammern (C1, C2) der Klammer (1) gebildete geschlossene Flüssigkeitskreis mit einer radioopaken Flüssigkeit gefüllt ist.

12. Eine teilweise Okkludiereinrichtung im Einklang mit Ansprüchen 1, 2, 3, 4, 5, 6, 7, 8, 9, 10 oder 11 mit einer Klammer (1), die aus zwei steifen oder halbsteifen U-förmig angeordneten Schenkeln (4, 5) mit einem gekrümmten Teil (6) besteht, so daß sie die Form einer Stimmgabel aufweist, wobei die besagte Einrichtung dadurch gekennzeichnet ist, daß sie ein Verschlußelement (8, 8') umfaßt, das an dem freien Ende eines Schenkels (5) gelenkig angeordnet und mit einer Einrasteinrichtung (8a) versehen ist, die in der Lage ist, mit einer entsprechenden Einrichtung (9) an dem freien Ende des anderen Schenkels (4) zusammenzuarbeiten, um die Klammer zu schließen.

13. Eine teilweise Okkludiereinrichtung gemäß Anspruch 12, dadurch gekennzeichnet, daß die Einrasteinrichtungen des Elements (8, 8') und des Schenkels so beschaffen sind, daß sie die beiden Schenkel so im Verhältnis zueinander blockieren, daß sie sich weder voneinander entfernen noch einander nähern können.

14. Eine teilweise Okkludiereinrichtung gemäß einem der Ansprüche 12 oder 13, dadurch gekennzeichnet, daß das Element (8) an dem Schenkel (5) mit Hilfe eines flexiblen Stegs (12) gelenkig angeordnet ist, der es verlängert und an dem besagten Schenkel angebracht ist.

15. Eine teilweise Okkludiereinrichtung gemäß einem der Ansprüche 12 oder 13, dadurch gekennzeichnet, daß das Element (8') ein Zusatzelement und an dem Schenkel (5) mit Hilfe einer Schale (22) und einer Drehspindel (23) gelenkig angeordnet ist.

16. Eine teilweise Okkludiereinrichtung gemäß einem der Ansprüche 12, 13, 14 oder 15, dadurch gekennzeichnet, daß das Element an seinem freien Ende eine flexible Positionierzunge (25) aufweist.

17. Eine teilweise Okkludiereinrichtung gemäß einem der Ansprüche 2, 3, 4, 5, 6, 7, 8, 9, 10 oder 11, mit einer Klammer (1), die aus zwei steifen oder halbsteifen U-förmig angeordneten Schenkeln (4, 5) und einem gekrümmten Teil (6) besteht, so daß sie die Form einer Stimmgabel aufweist, wobei die besagte Einrichtung dadurch gekennzeichnet ist, daß mindestens einer der Schenkel der Klammer im Bereiche seines freien Endes einen Zahn (24) umfaßt, der so beschaffen ist, daß er zum Anschwellen gebracht werden kann, um das Schließen der Klammer zu gewährleisten.

18. Eine teilweise Okkludiereinrichtung gemäß Anspruch 17, dadurch gekennzeichnet, daß die Schenkel (4, 5) der Klammer zwei einander gegenüberliegende Verschlußzähne (24) umfassen, die so beschaffen sind, daß sie beim Anschwellen aneinander anschließen.

19. Eine teilweise Okkludiereinrichtung gemäß einem der Ansprüche 17 oder 18, dadurch gekennzeichnet, daß jeder Verschlußzahn (24) aus einer Membran aus flexiblem Material besteht, die so beschaffen ist, daß sie dem Anschwellen nur einen vernachlässigbar geringen Widerstand bietet, während die die anderen Zähne (d) bildenden Membranen eine elastische Innenschicht umfassen, die in der Lage ist, dem Anschwellen einen Widerstand entgegenzusetzen, sowie eine nicht dehnbare Außenschicht, die so beschaffen ist, daß sie das Ausdehnungsvolumen der besagten Zähne begrenzt.

20. Eine teilweise Okkludiereinrichtung für Blutgefäße dadurch gekennzeichnet, daß sie einziehbare Zähne (d) umfaßt, die so beschaffen sind, daß sie zwischen einer aktiven Position, in der sie den inneren Hohlraum des Blutgefäßes in mehrere Kanäle unterteilen und einer passiven Position ohne merkbare Auswirkung auf das Blutgefäß verstellt werden können, wenn die besagte Klammer an der Außenseite des besagten Blutgefäßes angebracht ist.

21. Eine Klammer gemäß Anspruch 20, die aus zwei steifen oder halbsteifen U-förmig angeordneten Schenkeln (4, 5) und einem gekrümmten Teil (6) besteht, so daß sie die Form einer Stimmgabel aufweist, wobei die besagte Klammer dadurch gekennzeichnet ist, daß sie ein Verschlußelement (8, 8') umfaßt, das an dem freien Ende des Schenkels (5) gelenkig angeordnet und mit einer Einrasteinrichtung (8a) versehen ist, wobei diese in der Lage ist, mit einer entsprechenden Einrichtung (9) am freien Ende des anderen Schenkels (4) zusammenzuarbeiten, um die Klammer zu schließen.

22. Eine Klammer im Einklang mit Anspruch 20, die aus zwei steifen oder halbsteifen U-förmig angeordneten Schenkeln (4, 5) und einem gekrümmten Teil (6) besteht, so daß sie die Form einer Stimmgabel aufweist, wobei die besagte Klammer dadurch gekennzeichnet ist, daß mindestens einer der Schenkel der Klammer im Bereiche seines freien Endes einen Zahn (24) besitzt, der so beschaffen ist, daß er zum Anschwellen gebracht werden kann, um das Schließen der Klammer zu gewährleisten.

Fig. 1

Fig. 2

Fig. 3

Fig. 4

Fig. 5

Fig. 6a

Fig. 6b

Fig. 6c

Fig. 7

2

1

3

Fig. 8a

13

18

d

Fig. 8b

21

d

1

Fig. 9

25

Fig. 10

22

23

Fig. 11

22

23    8'

Fig. 12

d

24